(19) Europäisches Patentamt
European Patent Office
Office européen des brevets

(11) Numéro de publication: **0 177 376**
**B1**

# (12) FASCICULE DE BREVET EUROPÉEN

(45) Date de publication du fascicule du brevet: **12.10.88**

(51) Int. Cl.⁴: **C 07 C 143/155**, E 21 B 43/22, B 01 F 17/00

(21) Numéro de dépôt: **85401581.5**

(22) Date de dépôt: **02.08.85**

(54) **Composés tensio-actifs de la famille des sulfobétaines, leur préparation et leur application, notamment à la récupération assistée du pétrole.**

(30) Priorité: **20.08.84 FR 8413048**

(43) Date de publication de la demande:
**09.04.86 Bulletin 86/15**

(45) Mention de la délivrance du brevet:
**12.10.88 Bulletin 88/41**

(84) Etats contractants désignés:
**BE CH DE GB LI NL**

(56) Documents cité:
**US-A-4 259 191**

(73) Titulaire: **INSTITUT FRANCAIS DU PETROLE, 4, Avenue de Bois- Préau, F-92502 Rueil- Malmaison (FR)**
Titulaire: **CENTRE NATIONAL DE LA RECHERCHE SCIENTIFIQUE (CNRS), 15, Quai Anatole France, F-75700 Paris Cedex 07 (FR)**

(72) Inventeur: **Brunel, Sylvain, 6, rue Buffon, F-38200 Vienne (FR)**
Inventeur: **Germanaud, Laurent, 79, rue Route Neuve, F-69540 Irigny (FR)**
Inventeur: **Le Perchec, Pierre, 99, rue Pierre Corneille, F-69003 Lyon (FR)**
Inventeur: **Sillion, Bernard, 93, rue Juliot Curie, F-69005 Lyon (FR)**

(74) Mandataire: **Colas des Francs, Jean, Institut Français du Pétrole 4, Avenue de Bois Préau, F-92502 Rueil- Malmaison (FR)**

**EP 0 177 376 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1988

**Description**

La présente invention est relative à de nouveaux composés tensio-actifs du groupe des sulfobétaïnes, leur préparation et leur utilisation.

On sait que les tensio-actifs de la famille des sulfobétaïnes possèdent de bonnes propriétés complexantes vis-à-vis des ions divalents et de ce fait présentent un intérêt particulier pour la récupération des hydrocarbures par les techniques chimiques qui font appel aux systèmes micellaires.

L'objet de la récupération assistée du pétrole est d'augmenter son taux de récupération, d'une part grâce à la régularisation du balayage du gisement, d'autre part par la réduction suffisante des forces capillaires, notamment par l'abaissement de la tension interfaciale entre l'huile et le fluide injecté, qui permet d'augmenter l'efficacité du déplacement microscopique.

L'efficacité de balayage de l'injection d'eau est généralement améliorée par la réduction de la mobilité de l'eau obtenue par l'addition de polymères hydrosolubles. Plusieurs procédés sont proposés pour accroître l'efficacité de déplacement microscopique du fluide injecté: injection de solvants (gaz hydrocarbonés, gaz carbonique, alcools, gaz de pétrole liquéfié, etc), injection d'eau alcaline et de solutions d'agents tensio-actifs sous diverses formes: solutions aqueuses, microémulsions, etc. En ce qui concerne l'utilisation des tensio-actifs, habituellement combinée avec celle des polymères hydrosolubles pour stabiliser le déplacement, il est apparu que la récupération pouvait atteindre 50 % du volume de l'huile initiale, et même, dans les cas favorables, 70 %.

Une technique, désormais classique, pour abaisser la tension interfaciale entre l'huile du gisement et le fluide injecté consiste à injecter une solution d'un tensio-actif dont les caractéristiques sont choisies en fonction des conditions imposées par le gisement: notamment la salinité de l'eau, la nature de l'huile en place et de la roche et la température. Il est procédé ensuite à l'injection d'une solution de polymères hydrosolubles, suivie d'une injection d'eau.

Le composé tensio-actif est habituellement utilisé à une concentration supérieure à sa concentration micellaire critique. Les systèmes micellaires injectés sont soit des solutions aqueuses contenant des quantités variables de tensio-actifs et, éventuellement d'autres additifs, tels que cotensio-actif, cosolvant, électrolytes, etc, soit des mélanges en proportions variables d'eau, d'électrolytes, d'hydrocarbures et éventuellement de cotensio-actifs et/ou de cosolvant. Dans ce dernier cas, la présence de molécules polaires-apolaires, en concentration suffisante, conduit à la formation de mélanges transparents généralement dénommés microémulsions.

De très nombreux types de tensio-actifs ont été proposés dans l'art antérieur pour la récupération assistée du pétrole. Les tensio-actifs les plus couramment utilisés, pour des raisons de coût et de disponibilité, sont du type sulfonate, plus précisément des sulfonates de pétrole, présentés sous la forme de sels de métaux alcalins ou d'ammonium. L'emploi de ces tensio-actifs est satisfaisant tant que la teneur en sel de l'eau ne dépasse pas environ 30 g/l (en équivalent chlorure de sodium), cette valeur étant donnée pour situer l'ordre de grandeur: en particulier, les tensions interfaciales entre l'huile et les solutions de sulfonates, obtenues par le choix judicieux des caractéristiques du produit, sont très basses, de l'ordre de $10^{-3}$ mN/m, et même moins. Mais, lorsque la salinité dépasse sensiblement la valeur indiquée plus haut, il a été établi que les propriétés interfaciales des sulfonates se dégradent rapidement et ce d'autant plus que la teneur en cations divalents, calcium et magnésium notamment, est plus élevée. De plus, la grande sensibilité des sulfonates aux cations divalents provoque, au cours du cheminement de la solution de tensio-actif dans le réservoir, des phénomènes de précipitation et/ou de transfert du tensio-actif dans une phase immobile, phénomènes qui, liés au relargage de cations par la roche, tendent à rendre le tensio-actif inopérant.

Il a été proposé de substituer aux sulfonates de pétrole d'autres types de tensio-actifs anioniques: par exemple, des paraffines-sulfonates, des oléfines-sulfonates, des alkylsulfates, des alkylphosphates, des alcanoates, des N acyl α-aminoalcanoates, des carboxylates, sulfates et sulfonates d'alcools gras éthoxylés et d'alkylphénols échoxylés, etc, ainsi que des tensio-actifs non-ioniques: par exemple, des alcools gras éthoxylés, des alkylphénols éthoxylés, etc.

Malgré tout, ces tensio-actifs de remplacement des sulfonates de pétrole subissent une perte importante de leur efficacité interfaciale quand la salinité des eaux de gisement est élevée. Les tensio-actifs non-ioniques sont beaucoup moins sensibles que les tensio-actifs anioniques à la présence des cations divalents pour ce qui concerne les risques de précipitation. En revanche, leur défaut majeur réside en ce que leurs propriétés en solution (basse tension interfaciale notamment) sont très sensibles aux faibles variations de température. De plus, la répartition de ce type de produits (répartition liée à la polydispersité) entre les diverses phases liquides est telle qu'elle entraîne une diminution de sa concentration utile dans la solution. Enfin, le point de trouble apparaît à une température relativement basse.

Les mélanges de tensio-actifs anioniques et non-ioniques ont fait l'objet de nombreux travaux de laboratoire et les résultats montrent que de tels mélanges présentent des propriétés interfaciales intéressantes, même en présence d'ions divalents; cependant le risque en cas d'utilisation en milieu poreux est évidemment, celui de physi- ou chimisorption sélective qui modifierait rapidement la composition du mélange.

Les divers inconvénients présentés par les tensio-actifs usuels ont provoqué des recherches axées sur l'utilisation d'autres composés tensio-actifs, et plus particulièrement sur les composés de type zwitterionique, produits dont les propriétés de tensio-activité sont peu ou non affectées par la présence de cations polyvalents, et ceci dans des gammes étendues de température et de pH.

L'idée de fixer sur une même molécule tensio-active deux parties hydrophiles différentes a été développée dans différents laboratoires s'intéressant aux problèmes de récupération assistée. Par exemple, il a été étudié des alcools oxyéthylés modifiés en bout de chaîne hydrophile par un groupe sulfonique introduit par diverses techniques ainsi que des produits qui résultent de la modification d'alcool oxyéthylé par l'acide chloracétique.

Dans l'art antérieur, les tensio-actifs sulfobétaïniques sont obtenus d'une manière générale par condensation d'une amine tertiaire (dont un des groupements alkyle est constitué de 10 à 25 atomes de carbone) sur une sultone, propane-sultone ou butane-sultone, selon l'équation 1:

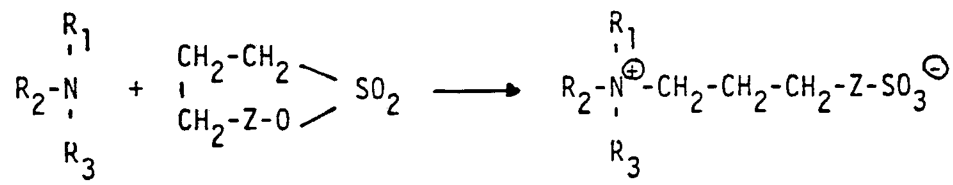

Equation 1

avec $Z = (CH_2)_n$ et $n = 0$ ou 1
on peut aussi opérer comme décrit par R.G. Bristline, W.R. Noble et W.M. Linfield dans J. Amer. Oil Chem. S. 53, 64, 1976, en faisant réagir sur les mêmes amines tertiaires le produit de condensation de l'épichlorhydrine sur le bisulfite de sodium, selon l'équation 2:

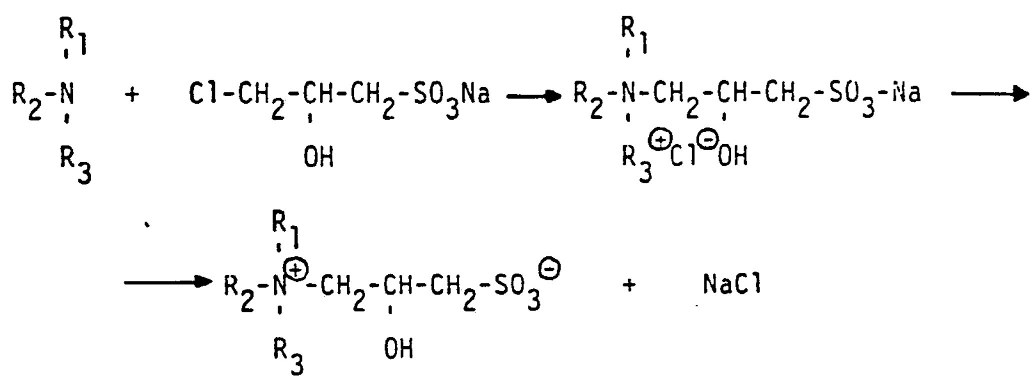

Equation 2

on obtient ainsi des sulfobétaïnes hydroxylées qui présentent une solubilité améliorée.

Les mêmes auteurs signalent que la quaternisation de l'amine tertiaire par le chlorure d'allyle, suivie de l'addition de bisulfite de sodium conduit aussi aux sulfobétaïnes selon l'équation 3:

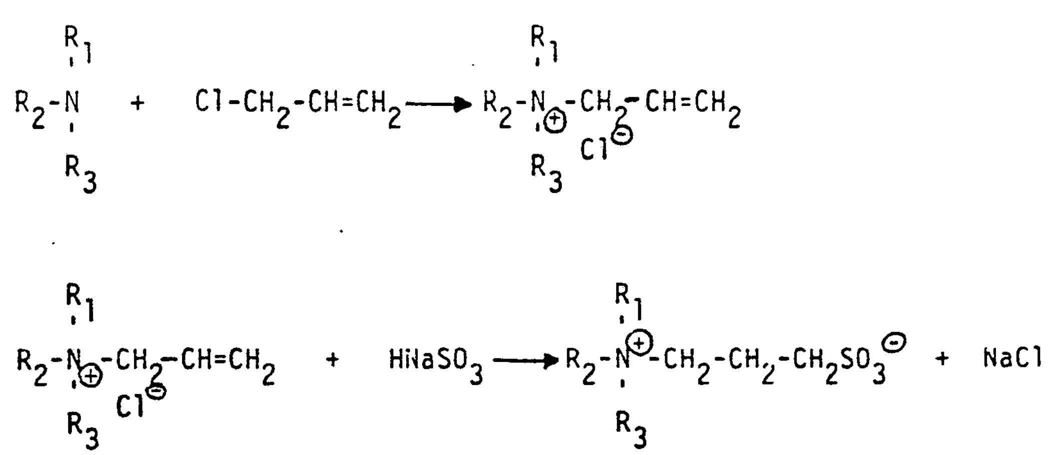

Equation 3

La condensation des esters de l'acide éthène sulfonique sur les sels d'amines tertiaires a été décrite dans le

brevet français 2 270 241 pour préparer les sulfobétaïnes selon l'équation 4:

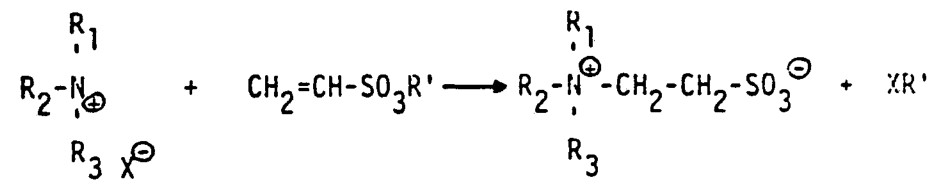

Equation 4

En outre, certains travaux proposent la synthèse de sulfobétaïnes de solubilité améliorée par introduction de fonctions hydrophiles telles que les groupes amides. A cet égard, le brevet US 4 259 191 décrit des produits issus de la réaction de propane-sultones sur des amides-amines dérivées des acides naphténiques, représentée par l'équation 5.

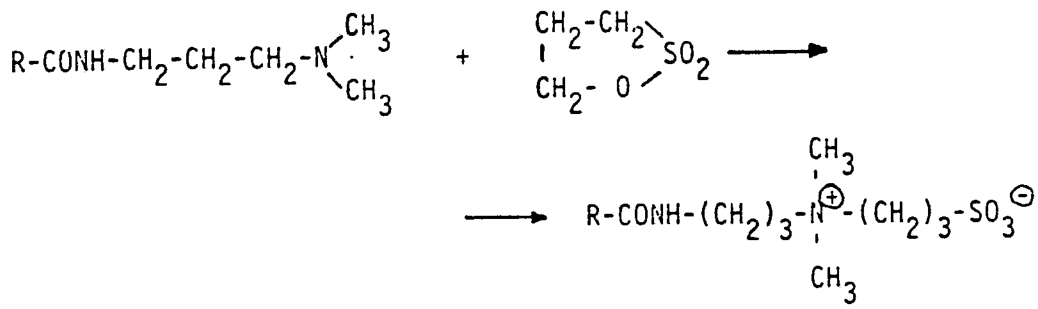

Equation 5.

dans lequel R représente un reste d'acide naphténique.

Par ailleurs, les propriétés tensio-actives des sulfobétaïnes et leurs excellentes propriétés dans les eaux dures ont déjà été mises en évidence et signalées dans plusieurs documents, comme par exemple l'article de W.R. Noble et W.M. Linfield, J.A.O.C. 57, 368, 1980 et celui de G.W. Fernley, J.A.O.C., 55, 98, 1978.

Leur utilisation pour les gisements salins, contenant notamment des ions divalents a déjà été décrite antérieurement. Le brevet US 4 216 097 décrit l'utilisation d'un produit répondant à la formule:

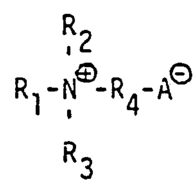

avec $R_4$ contenant de 1 à 6 carbones $A^{\ominus}$ étant $COO^{\ominus}$ ou $SO_3^{\ominus}$, $R_1$ étant la chaîne lipophile, mais parmi les composés cités, la chaîne $R_4$ la plus longue comprend un groupement méthylbutylène. Dans ce brevet, des indications intéressantes sont données sur l'efficacité à très faible concentration de ce type de produit et sur leur faible adsorption, en particulier, il est fait mention dans le cas du produit $C_{16}H_{33}N^{+}(CH_3)_2$-$CH_2$-$CH_2$-$SO_3^{-}$ d'une excellente récupération de pétrole dans un milieu à forte concentration en ions divalents.

Dans leur ensemble, les sulfobétaïnes de l'art antérieur possèdent une distance entre les deux pôles du zwitterion qui est déterminée par la nature du réactif sulfonique et cette distance est généralement limitée à quatre atomes de carbone en chaîne linéaire.

La présente invention montre qu'il est possible de préparer des sulfobétaïnes dont la distance entre les charges est supérieure à la distance inhérente à la présence de 4 atomes en chaîne linéaire, ce qui leur confère des propriétés physiques (la solubilité par exemple) et des propriétés tensio-actives améliorées.

Les sulfobétaïnes de l'invention peuvent être représentées par la formule générale:

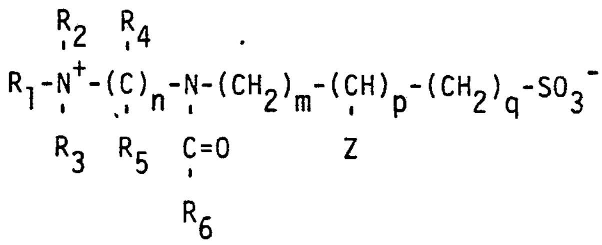

(I)

dans laquelle $R_1$, $R_2$ et $R_3$ représentent chacun un radical aliphatique linéaire ou ramifié, porteur ou non de fonctions hydroxyles, ou bien un radical aromatique ou arylaliphatique, $R_4$ et $R_5$ sont chacun un atome d'hydrogène, un groupe méthyle ou un groupe hydroxyméthyle et $R_6$ est un atome d'hydrogène, un groupe alkyle linéaire ou ramifié ou un groupe aryle, l'ensemble des radicaux $R_1$ à $R_6$ renfermant de 12 à 30 atomes de carbone, n est un nombre entier égal à 2 ou à 3, Z peut être un radical $CH_3$ ou un groupement hydroxyle, p peut être égal à 0 ou à 1 avec, lorsque Z est un groupement hydroxyle, p étant égal à 1, m est égal à 1 et q est égal à 1; lorsque Z est un radical méthyle, p étant égal à 1, m est égal à 2 et q peut prendre la valeur 0 ou 1; et si p = 0, la somme (m + q) est égale à 2, 3 ou 4.

La distance séparant les deux pôles du zwitterion peut donc être de 5 à 8 atomes en chaîne linéaire (carbone + azote).

L'un des trois radicaux renferme avantageusement au moins 10 atomes de carbone, les deux autres renferment un nombre d'atomes de carbone de 1 ou 2; $R_4$ et $R_5$ sont avantageusement des atomes d'hydrogène et $R_6$ est avantageusement un atome d'hydrogène ou un groupe alkyle linéaire ou ramifié, renfermant de 1 à 20 atomes de carbone, ou encore un groupe aryle.

Si la longueur de la chaîne est apportée par l'ensemble des groupes $R_1$, $R_2$ et $R_3$, le groupe $R_6$ renfermera avantageusement un nombre d'atomes de carbone de 1 à 6; ce sera de préférence un groupe méthyle.

Parmi les sulfobétaïnes définies ci-dessus, on considère comme particulièrement intéressantes celles de formule (I) dans lesquelles $R_1$ est un radical aliphatique saturé linéaire de $C_{12}$ à $C_{18}$, $R_2$ et $R_3$ sont des radicaux méthyles, $R_4$ et $R_5$ sont des atomes d'hydrogène, $R_6$ est un radical méthyle, n est égal à 2 ou 3, p est nul et (m + q) est égal à 3 ou 4.

Les sulfobétaïnes de l'invention, répondant à la formule (I) sont obtenues en deux étapes principales à partir des iminoéthers cycliques de formule générale:

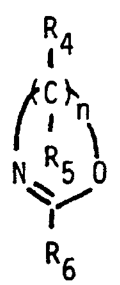

(II)

dans laquelle $R_4$, $R_5$, $R_6$ et n peuvent prendre les valeurs décrites pour la formule générale (I).

Les iminoethers cycliques utilisables dans le cadre de l'invention sont par exemple la méthyl-2, 2-oxazoline-1,3, la phenyl-2, 2,-oxazoline-1,3, la méthyl-2 diméthyl-4,4, 2-oxazoline-1,3, la méthyl-2 diméthyl-5,5, 2-oxazoline-1,3, la méthyl-2, méthyl-4 hydroxymethyl-4, 2-oxazoline-1,3, la méthyl-2, bishydroxyméthyl-4,4, 2-oxazoline-1,3, l'hexyl-2, 2-oxazoline-1,3, la nonyl-2, 2-oxazoline-1,3, la méthyl-2, dihydro-5,6 2-oxazine-1,3, la nonyl-2, dihydro-5,6 2-oxazine-1,3 et la phenyl-2, dihydro-5,6 2-oxazine-1,3.

Les sultones utilisables dans le cadre de l'invention sont par exemple: propane sultone, butane sultone, méthyl-4 butane sultone et méthyl-3 propane sultone.

Les iminoéthers cycliques (II) utilisés au départ du procédé de préparation des produits de l'invention peuvent être préparés par des méthodes connues qui font appel:

- soit à la condensation des nitriles sur les aminoalcools-1,2 ou-1,3, catalysée par les sels de cadmium, comme décrit par W. Seeliger et coll., Angew. Chem. Intern. Ed. 5, (10), 875, 1966.

- soit au réarrangement d'acylaziridine, par exemple, selon la méthode décrite par A.I. Meyer et coll., J. Org. Chem. 39, (18), 2787, 1974.

Dans la première étape, l'iminoéther cyclique (II) est transformé en une sulfobétaïne intermédiaire selon l'une ou l'autre des méthodes suivantes: selon un premier mode de réalisation de cette première étape, on condense sur l'iminoéther (II) une sultone de formule générale:

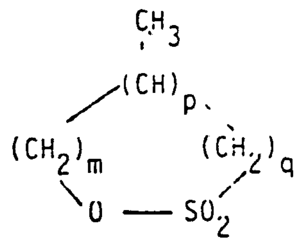

(III)

dans laquelle, lorsque p = 0, (m + q) est égal à 3 ou 4 et lorsque p = 1, m est égal à 2 et q est égal à 0 ou 1, selon l'equation 6:

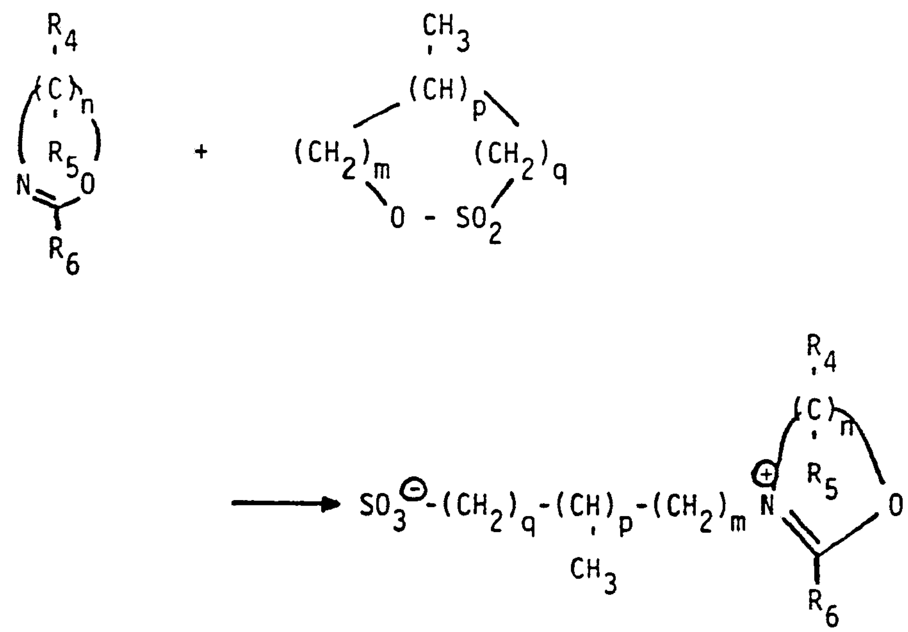

Equation 6

dans laquelle m, p et q sont définis comme dans la formule (III).

Les conditions de cette réaction sont en général les suivantes:

On opère préférentiellement en solution 10 à 30 % molaire des réactifs en proportions stoechiométriques dans un solvant chloré par exemple le chlorobenzène. La température de réaction peut être comprise entre 20 et 70° C. La durée de réaction est comprise entre 1 heure et 24 heures.

La première étape du procédé de préparation peut également mettre en jeu le produit de la réaction du bisulfite de sodium sur l'épichlorhydrine, que l'on condense sur l'iminoéther (II) selon l'équation 7.

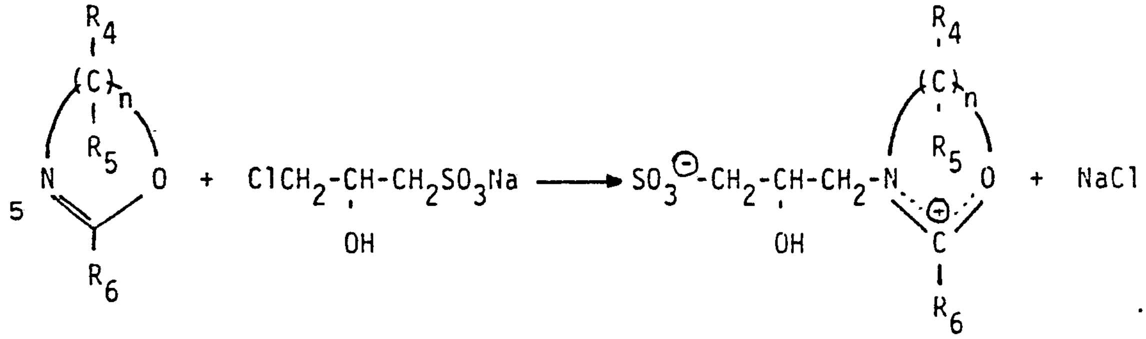

Equation 7

La réaction s'effectue dans les conditions stoechiométriques, en milieu biphasique (eau et hydrocarbure) et à une température comprise entre 20 et 80° C.

La première étape du procédé de préparation peut aussi consister en la condensation d'un ester de l'acide éthène sulfonique sur un halohydrate ou un hydrogénosulfate d'iminoéther de formule IIa, selon l'équation 8.

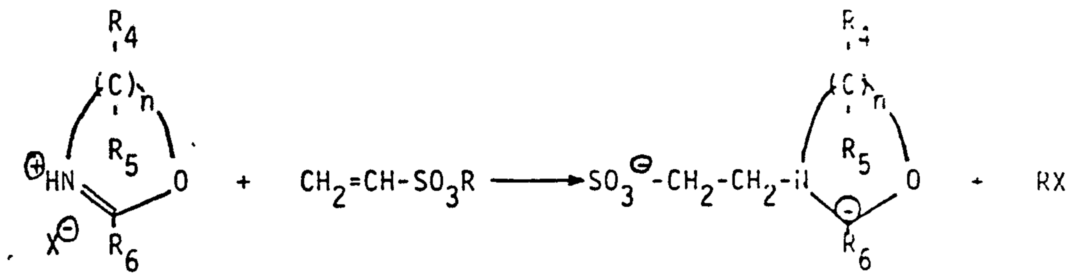

(IIa)

Equation 8

dans laquelle R est un radical aliphatique comprenant de 1 à 10 atomes de carbone.

La préparation des produits de l'équation 8 est inspirée des publications de A. Le Berre, A. Etienne, A. Delacroix et A. Proust, Bull. Soc. Chim., 1975, n° 11, page 2531 et Bull. Soc. Chim., 1973 n° 7, page 2404.

Selon un autre mode de mise en oeuvre de la première étape du procédé de préparation, on peut condenser l'iminoéther (II) sur le chlorure d'allyle puis le chlorure d'iminoéther cyclique intermédiaire est condensé sur le bisulfite de sodium selon l'équation 9.

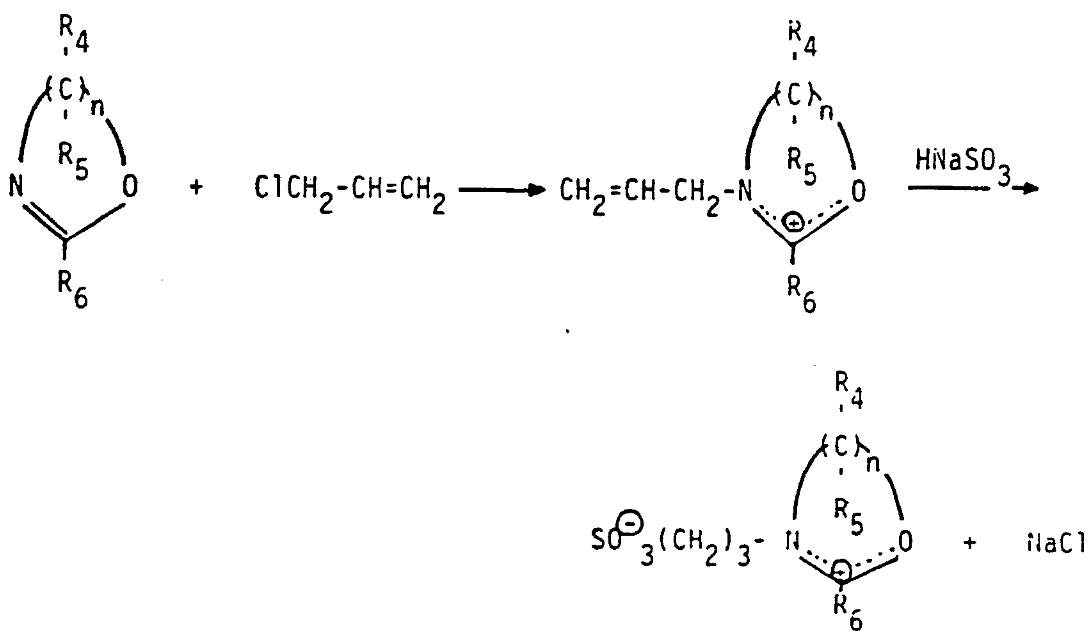


Equation 9

La première réaction s'effectue à la Température ambiante, dans des conditions stoechiométriques et en présence d'un solvant hydrocarboné tandis que la condensation sur le bisulfite de sodium s'effectue en milieu biphasique dans les conditions stoéchiometriques et à une température entre 20 et 80° C.

L'une ou l'autre des méthodes indiquées ci-dessus conduit à une sulfobétaïne intermédiaire de formule générale:

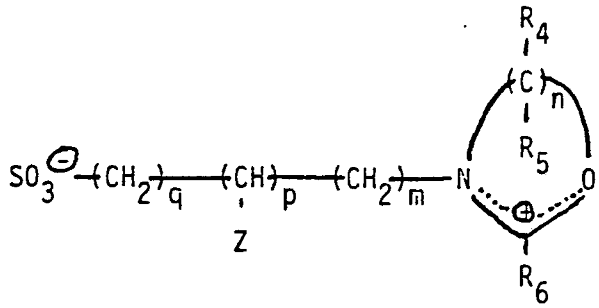

(IV)

dans laquelle $R_4$, $R_5$, Z, n, m, p, q et Z ont les significations indiquées précédemment pour la formule générale I.

Dans la deuxième étape, les sulfobétaïnes intermédiaires (IV) sont alors condensées sur des amines tertiaires de formule générale:

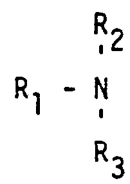

(V)

dans laquelle $R_1$, $R_2$, $R_3$ ont les valeurs indiquées pour la formule (I); cette condensation conduit directement aux sulfobétaïnes (I) de l'invention.

Les amines utilisables dans le cadre de l'invention sont par exemple: la N,N-diméthyl octylamine, la N,N-diméthyl décylamine, la N,N-diméthyl dodecylamine, la N, N-diméthyl octadécylamine, les mélanges industriels issus de la méthylation des amines grasses du coprah, du suif, la N,N-bis (hydroxyéthyl) dodécylamine et la N,N-diéthyl benzylamine.

Les sulfobétaïnes de l'invention, telles qu'elles ont été difinies précédemment, peuvent être avantageusement utilisées comme tensio-actifs dans la récupération assistée du pétrole.

Les systèmes micellaires considérés peuvent être définis de manière générale par le fait qu'il contiennent, à titre de tensio-actif, au moins une sulfobétaïne selon l'invention, en mélange avec de l'eau, éventuellement au moins un liquide hydrocarboné, et éventuellement au moins un cotensio-actif. On peut les mettre en jeu comme produits purs ou sous la forme de solutions aqueuses préalablement formées, de telles solutions pouvant avoir par exemple des concentrations d'environ 1 à 40 % en poids de matière active.

Les systèmes micellaires considérés dans l'invention peuvent consister en des solutions aqueuses de concentrations en produits tensio-actifs variées, allant par exemple de 0,1 à 15, en poids.

Dans ces solutions, on peut introduire en outre, au moins un cotensio-actif ou un cosolvant tel que défini ci-après en une proportion pouvant aller par exemple jusqu'à 15 % en poids par rapport au poids total de la solution.

Les cotensio-actifs ou "cosolvants" sont principalement des alcools, notamment des mono-alcools aliphatiques primaires de 1 à 12 atomes de carbone. Parmi ceux-ci, on utilisera avantageusement: le n-propanol, l'isobutanol, le n-butanol-1, le n-pentanol-1, le n-hexanol-1, le n-heptanol-1, le n-octanol-1, le n-décanol-1 ou le n-dodécanol-1, seuls ou en mélanges entre eux. Comme cotensio-actifs on peut encore utiliser des amines, des acides, des éthers, des polyols, ainsi que des tensio-actifs non ioniques, tels que des éthoxylats d'alcools gras, d'acides gras ou d'alkylphénols, ou encore des tensio-actifs anionique tels que des composés portant des fonctions sulfate, sulfonate, carboxylate ou phosphonate. Ces divers cotensio-actifs peuvent être utilisés seuls ou en mélange entre eux.

Le tensio-actif et le cotensio-actif ou le cosolvant peuvent être entre eux dans des rapports variés; avantageusement, le rapport pondéral du cotensio-actif ou du cosolvant au tensio-actif sera compris entre 0 et 5/1 et de préférence de 1/1 à 3/1.

L'eau utilisée pour préparer ces solutions, qui peut éventuellement consister en de l'eau de gisement, peut contenir des cations mono/et ou polyvalents, en particulier $Na^+$, $K^+$, $Ca^+$, et $Mg^{++}$ (la concentration saline totale pouvant être par exemple de 30 à 300 g/l). La proportion d'eau va de 70 à 99,9 %.

Les systèmes micellaires de l'invention peuvent également consister en des microémulsions comprenant:

- de l'eau, qui, comme indiqué ci-dessus, peut contenir des cations mono et/ou polyvalents, en particulier $Na^+$, $K^+$, $Ca^{++}$, ou $Mg^{++}$ (la concentration saline totale pouvant être par exemple de 30 à 300 g/l);

- au moins un liquide hydrocarboné, qui peut être un hydrocarbure pur, renfermant par exemple de 8 à 16 atomes de carbone, un mélange d'hydrocarbures, une coupe de fractionnement de pétrole ou encore un pétrole brut;

- au moins une sulfobétaïne telle que décrite précédemment; et
- éventuellement au moins un cotensio-actif (ou cosolvant) tel que défini précedemment.

Les microémulsions peuvent comprendre des proportions variées des divers constituants, par exemple:
- de 70 à 99,9 en poids d'eau et de liquide hydrocarboné dans un rapport pondéral liquide hydrocarboné/eau de 1/100 à 4/1 et de préférence de 1/20 à 1/1,
- de 0,1 à 15 % en poids de tensio-actif (sulfobétaïne),
- de 0 à 15 % en poids de cotensio-actif.

Les exemples suivants illustrent l'invention et ne doivent en aucune manière en limiter la portée.

**Exemple 1:** Préparation du (méthyl-2 oxazolinium-1,3)-3 propane sulfonate selon A. Forestiere et B. Sillion, J. Heterocyclic Chem. 17, 1381 (1980).

On laisse réagir sous agitation à temperature ambiante une solution de 16 g (0,131 mole) de propanesultone-1,3 et de 11,2 g (0,131 mole) de méthyl-2 oxazoline-1,3 dans 50 ml de chlorobenzène sous atmosphère inerte. (Il est impératif que tous les réactifs soient fraîchement distillés). Après 24 h, la bétaïne a précipité et on additionne 50 ml de cyclohexane anhydre, essore les cristaux sous gaz inerte, puis les lave au cyclohexane et les séche sous bon vide. On obtient 23,6 g (87 %) de cristaux blancs très hygroscopiques (F = 236°) identifiés par spectrométrie I.R. et R.M.N. du proton. La microanalyse correspond à la théorie.

**Exemple 2:** Préparation de l'acétyl-4 aza-4 (N,N-diméthyl-laurylammonium)-6 hexane sulfonate.

On chauffe à 120° sous agitation pendant 3 heures le mélange de 23,5 g (0,113 mole) de la bétaïne de l'exemple 1,33,6 ml (26,7 g ; 0,125 mole) de N,N-diméthyl-laurylamine distillée dans 70 ml de diméthyl formamide (DMF) anhydre. On évapore le solvant, reprend par 300 ml d'eau permutée, lave par deux fois 150 ml de chloroforme (pour éliminer l'excès d'amine) puis concentre à sec. Un ajoute puis évapore à sec trois fois 500 ml de méthanol sec puis sèche sous bon vide. Le produit hygroscopique, est conservé sous argon. On a 40 g (89 %) de produit (huile vitrifiée) dont la pureté (95 %) est vérifiée par chromatographie liquide haute performance (HPLC). Les spectres IR et $^1H$ RMN confirment la structure et montrent qu'il reste un peu d'eau; les résultats de la micro-analyse indiquent la présence de 0,25 mole d'eau par mole de sulfobétaïne.

**Exemple 3:** Préparation de l'acétyl-4 aza-4 (N,N-diméthyl stearylammonium)-6 hexane sulfonate.

On chauffe à 120° pendant 3 heures, en l'agitant, un mélange de 8,1 g (0,0391 mole) du sel de l'exemple 1 et 12 g (0,0405 mole) de N,N-diméthyl stéarylamine dans 40 ml de DMF anhydre. On laisse revenir à température ambiante, ce qui donne lieu à une prise en masse du milieu réactionnel, auquel on doit ajouter 100 ml d'acétone pour pouvoir isoler les cristaux par essorage sous gaz inerte, lavage à l'acétone et séchage. On obtient 13 g (65 %) de cristaux blancs très hygroscopiques conservés sous argon. La structure est clairement établie par spectrométrie IR et $^1H$ RMN, et un échantillon recristallisé dans un mélange acétone-acétonitrile (9/1 en volume) présente une micro-analyse en accord avec la formule brute.

**Exemple 4:** Préparation du (méthyl-2 dihydro-5,6 oxazinium-1,3)-3 propane sulfonate.

On laisse réagir sous agitation, à température ambiante et sous atmosphère inerte, la solution de 9 g (0,09 mole) de méthyl-2 dihydro-5,6 oxazine, 10,8 g (0,088 mole) de propane sultone-1,3 fraichement distillée, dans 35 ml de chlorobenzène anhydre. Après 24 heures, on ajoute 50 ml de cyclohexane sec, essore les cristaux, les lave au cyclohexane puis à l'acétone et sàche sous bon vide. On obtient 16,35 g (84 %) de cristaux blancs très hygroscopiques identifiés par $^1H$ RMN.

**Exemple 5:** Préparation de l'acétyl-4 aza-4 (N,N-diméthyl laurylammonium)-7 heptane sulfonate.

On chauffe à 120°, en agitant et sous atmosphère inerte, pendant 3 h. le mélange de 16,3 g (0,074 mole) de bétaïne (ex. 4), 22,5 ml (17,8 g; 0,083 mole) de N,N-diméthyl laurylamine (distillée) dans 45 ml de DMF anhydre. On évapore le solvant, reprend par 100 ml d'eau permutée, lave par 2 x 50 ml de chloroforme (élimination de l'amine résiduelle) et ajoute puis évapore 3 x 250 ml de méthanol sec, et sàche sous bon vide. On obtient 30,7 g (93 %) de produit solide (huile vitrifiée) hygroscopique que l'on conserve sous argon.

La structure est vérifiée par spectrométrie IR et $^1H$ RMN montrant une présence d'eau. La pureté, déterminée par HPLC est de 100 % et la micro-analyse donne des résultats en accord avec la structure en tenant compte de la présence de 0,75 molécule d'eau par mole de sulfobétaïne.

**Exemple 6:** Préparation du (méthyl-2 dihydro-5,6 oxazinium-1,3)-4 butane sulfonate.

On chauffe à 70°C en agitant sous atmosphère inerte et pendant 24 heures le mélange de 13,6 g (0,1 mole) de butane sultone-1,4 et 10 g (0,1 mole) de méthyl-2 dihydro-5,6 oxazine dans 50 ml de chlorobenzène anhydre. On laisse refroidir et obtient deux phases liquides troubles. On élimine la phase surgageante et additionne de l'acétone sèche qui fait cristalliser le produit. On essore, lave à l'acétone et sèche la pâte très hygroscopique obtenue (12 g; 51 %) dont la structure est vérifiée par RMN du proton.

**Exemple 7:** Préparation de l'acétyl-5 aza-5 (N,N-diméthylstéarylammonium)-8 octane sulfonate.

On agite sous gaz inerte pendant 24 heures à 120°C le mélange du produit obtenu en exemple 6 (0,05 mole), 16,3 g (0,055 mole) de N,N-diméthylstéarylamine dans 40 ml de DMF anhydre. On évapore en partie la DMF sous bon vide, additionne de l'acétone anhydre, filtre sous atmosphère inerte et séche sous bon vide. On obtient 15,8 g (59 %) de cristaux beiges qui sont recristallisés dans un mélange acétone-méthanol (99/1 volume) pour donner 15,5 g (58 %) de cristaux blancs caractérisés par spectrométrie IR et $^1H$ RMN (présence d'eau). La micro-analyse est correcte en tenant compte de la présence de 0,25 molécule d'eau par mole de sulfobétaïne.

**Exemple 8:** Solubilité dans l'eau à 20°C des sulfobétaïnes des exemples 2, 3, 5 et 7 (Tableau I).

| FORMULE | Exemple | Solubilité (g/l) |
|---|---|---|
| 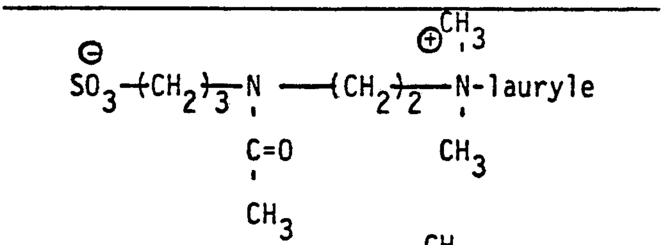 | 2 | 300 |
| 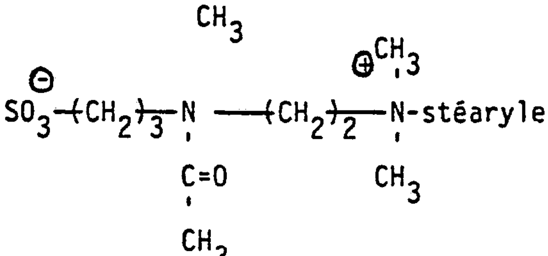 | 3 | 80 |
| 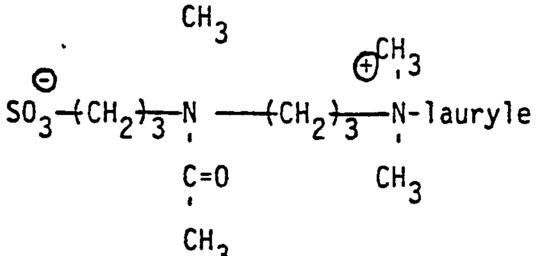 | 5 | 300 |
| 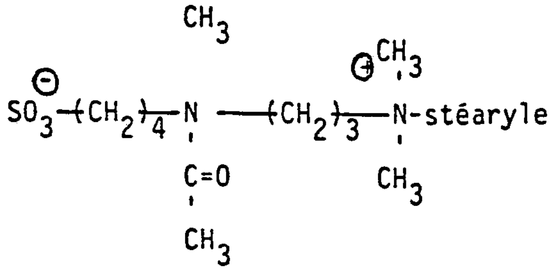 | 7 | 4 |

**Exemple 9:** Mesure de concentration micellaire critique (CMC) des sulfobétaïnes des exemples 2, 3, 5 et 7.

On définit la concentration micellaire critique d'un amphiphile comme la concentration minimale au-dessous de laquelle la concentration en micelle devient nulle ainsi que toutes les propriétés qui s'y rattachent (indice de refraction, densité, conductivité spécifique, turbidité, coefficient osmotique, tension superficielle et solubilité d'un solvant insoluble dans l'eau) selon R. J. Williams et al., Trans. Faraday Soc. 1968.

Préparation des solutions pour la mesure des CMC.

On utilise une méthode standard qui utilise des solutions de concentration initiale de 0,5 g/l de tensio-actif (TA) dans l'eau fraîchement permutée à la température ambiante mesurée 20°C. Le mélange TA-eau est agité jusqu'à dissolution totale puis une série de solutions titrées sont obtenues par dilution successive en prélevant un volume déterminé de solution-mère ramené à chaque fois à un volume standard, soit 1 ml + 39 ml; 2 ml +

38 ml; 3 ml + 37 ml; ... On agite 30 minutes les diverses solutions et laisse reposer 2 heures. Les mesures de tensions superficielles sont réalisées à l'aide d'un tensiomètre Tensiomat n3 Prolabo dont le principe consiste à mesurer la force d'arrachement d'un étrier placé à l'interface au/TA-air ambiant exprimée en millinewton/mètre.

La CMC est lue au point d'inflexion des deux droites obtenues.

Les valeurs sont résumées dans le tableau II

| FORMULE | EXEMPLE | CMC (g/l) | tension superficielle (mN/m) |
|---|---|---|---|
| 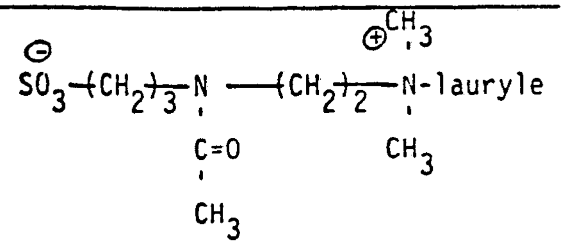 | 2 | 0,04 | 54 |
| 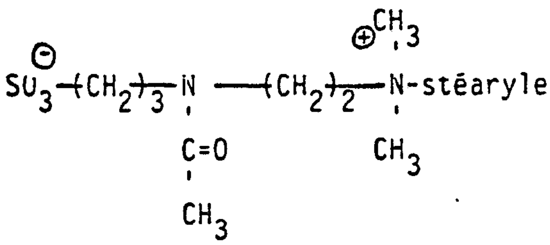 | 3 | 0,01 | 35 |
| 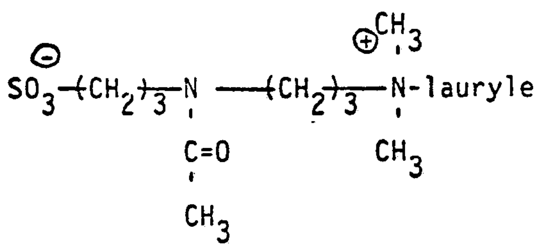 | 5 | 0,15 | 49 |
| 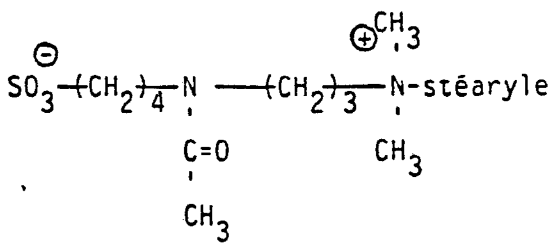 | 7 | 0,0028 | 39 |

**Exemple 10:** Test de micro-émulsion: Détermination des paramètres de solubilité optimale et de salinité optimale.

Les propriétés tensio-actives ont été déterminées à partir de l'analyse du comportement des sulfobétaïnes dans un mélange défini selon les conditions standard:

- saumure ($NaCl$, $CaCl_2$ avec $\frac{NaCl}{CaCl_2} = \frac{9}{1}$): 4,5 g
- tensio-actif: 500 mg
- cotensio-actif: 500 mg
- huile: 4,5 g.

Selon la salinité de la saumure (0-200 g/l), on peut observer trois systèmes mono-di ou triphasiques si le produit présente des propriétés tensio-actives:

1) Le comportement défini par l'appellation Windsor I pour lequel on observe un équilibre entre phase huile

et phase µε*.

2) Un comportement défini par l'appellation Windsor II pour lequel on observe un équilibre entre phase µε et phase aqueuse.

3) Et enfin un système dit Windsor III dans lequel la phase µε intermédiaire est en équilibre avec une phase huileuse surnageante et une phase aqueuse.

* microémulsion

Ce dernier système offre la particularité de présenter des tensions interfaciales très basses. A ce titre, il est recherché pour son efficacité dans le dépiégeage des gouttelettes retenues par les forces capillaires. On définit dans ce système les paramètres de solubilité optimale et de salinité optimale qui correspondent à une égale quantité d'eau et d'huile dans la micro-émulsion.

A titre d'exemple pour la sulfobétaïne:

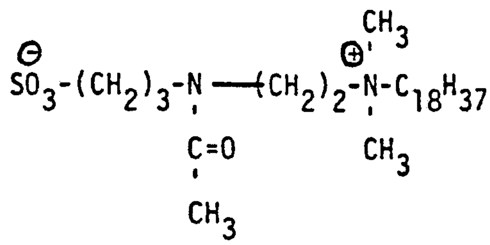

les paramètres suivants ont été obtenus avec le pentanol comme cotensio-actif et le dodécane comme huile

- paramètre de salinité optimale: 30 g/l
- paramètre de solubilité optimale: 55 %.

**Revendications**

1. Sulfobétaïne caractérisée en ce qu'elle répond à la formule générale:

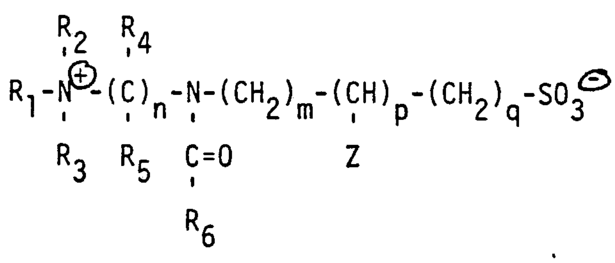

(I)

dans laquelle $R_1$, $R_2$ et $R_3$ représentent chacun un radical aliphatique linéaire ou ramifié, porteur ou non de fonctions hydroxyles, ou bien un radical aromatique ou arylaliphatique, $R_4$ et $R_5$ sont chacun un atome d'hydrogène, un groupe méthyle ou un groupe hydroxyméthyle et $R_6$ est un atome d'hydrogène, un groupe alkyle linéaire ou ramifié ou un groupe aryle, l'ensemble des radicaux $R_1$ à $R_6$ renfermant de 12 à 30 atomes de carbone, n est un nombre entier égal à 2 ou à 3, Z peut être un radical $CH_3$ ou un groupement hydroxyle, p peut être égal à 0 ou à 1 avec, lorsque Z est un groupement hydroxyle, p étant égal à 1, m est égal à 1 et q est égal à 1; lorsque Z est un radical méthyle, p étant égal à 1, m est égal à 2 et q peut prendre la valeur 0 ou 1; et si p = 0, la somme (m + q) est égale à 2, 3 ou 4.

2. Sulfobétaïne selon la revendication 1 caractérisée en ce que l'un des trois radicaux $R_1$, $R_2$ et $R_3$ renferme au moins 10 atomes de carbone, les deux autres renferment un nombre d'atomes de carbone de 1 ou 2, $R_4$ et $R_5$ sont des atomes d'hydrogène et $R_6$ est un atome d'hydrogène, un groupe alkyle linéaire ou ramifié renfermant de 1 à 20 atomes de carbone ou un groupe aryle.

3. Sulfobétaïne selon la revendication 1, caractérisée en ce que l'un des trois radicaux $R_1$, $R_2$ et $R_3$ renferme au moins 10 atomes de carbone, les deux autres renferment un nombre d'atomes de carbone de 1 ou 2 et $R_6$ est un radical alkyle de 1 à 6 atomes de carbone.

4. Sulfobétaïne selon l'une des revendications 1 à 3, caractérisée en ce que $R_1$ est un radical aliphatique saturé linéaire de $C_{12}$ à $C_{18}$, $R_2$ et $R_3$ sont des radicaux méthyle, n est égal à 2 ou 3, p est nul et (m + q) est égal à 3 ou 4.

5. Procédé de préparation d'une sulfobétaïne selon l'une des revendications 1 à 4, caractérisé en ce que dans une première étape, on prépare une sulfobétaïne intermédiaire de formule générale:

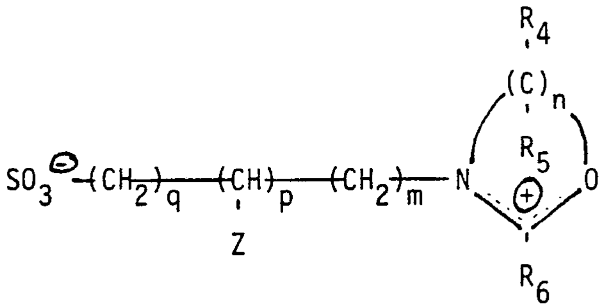

(IV)

dans laquelle $R_4$, $R_5$, $R_6$, n, m, p, q et Z sont définis comme dans la formule (I); dans une deuxième étape, on fait réagir ladite sulfobétaïne intermédiaire avec une amine tertiaire de formule générale:

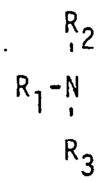

(V)

dans laquelle $R_1$, $R_2$ et $R_3$ sont définis comme dans la formule (I).

6. Procédé selon la revendication 5, caractérisé en ce que dans la première étape, on prépare une sulfobétaïne intermédiaire de formule générale (IV), dans laquelle Z est un radical méthyle, avec lorsque p = 0, m + q = 3 ou 4 et avec, lorsque p = 1, m est égal à 2 et q est égal à 0 ou 1, en faisant réagir un iminoéther cyclique de formule générale:

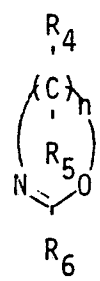

(II)

où $R_4$, $R_5$, $R_6$ et n sont définis comme dans la formule (I), sur de la propane sultone, de la butane sultone, de la méthyl 3 propane sultone ou de la méthyl 4 butane sultone.

7. Procédé selon la revendication 5, caractérisé en ce que dans la première étape, on prépare une sulfobétaïne intermédiaire de formule générale (IV) dans laquelle, lorsque Z est un groupement hydroxyle, m, p et q ont chacun la valeur 1, en faisant réagir un iminoéther cyclique de formule générale:

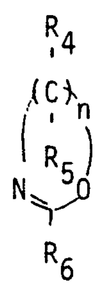

(II)

où $R_4$, $R_5$, $R_6$ et n sont définis comme dans la formule (I), avec le produit de réaction du bisulfite de sodium sur l'épichlorhydrine.

8. Procédé selon la revendication 5, caractérisé en ce que dans la première étape, on prépare une sulfobétaïne intermédiaire de formule générale (IV) dans laquelle p = 0 et m + q = 2, en faisant réagir un halohydrate ou un hydrogénosulfate d'iminoéther cyclique de formule générale (IIa)

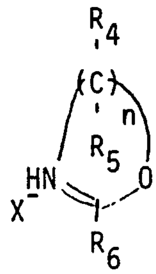

(IIa)

où X- représente un ion hydrogénosulfate ou halohydrate et, où $R_4$, $R_5$, $R_6$ et n sont définis comme dans la formule (I), avec un ester de l'acide éthène sulfonique de formule $CH_2 = CH - SO_3R$ dans lequel R est un radical aliphatique comprenant de 1 à 10 atomes de carbone.

9. Procédé selon la revendication 5, caractérisé en ce que dans la première étape, on prépare une sulfobétaïne intermédiaire de formule générale (IV), dans laquelle $p = 0$ et $m + q = 3$, en faisant réagir un iminoéther cyclique de formule générale:

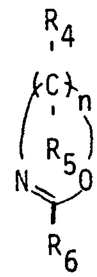

(II)

où $R_4$, $R_5$, $R_6$ et n sont définis comme dans la formule (I), avec du chlorure d'allyle, puis en faisant réagir le produit obtenu sur du bisulfite de sodium.

10. Système micellaire pour la récupération assistée du pétrole dans des gisements, caractérisé en ce qu'il contient à titre de tensio-actif, une sulfobétaïne conforme à l'une des revendications 1 à 4 en mélange avec de l'eau, éventuellement au moins un liquide hydrocarboné et éventuellement au moins un cotensio-actif.

11. Système micellaire selon la revendication 10, consistant en une solution micellaire ou une microémulsion, caractérisé en ce qu'il contient:

- de 70 à 99,9 % en poids d'un liquide hydrocarboné et d'eau dans un rapport pondéral liquide hydrocarboné/eau de 0 à 4/1,
- de 0,1 à 15 % en poids de sulfobétaïne et,
- de 0 à 15 % en poids de cotensio-actif.

12. Système micellaire selon l'une des revendications 10 et 11, caractérisé en ce que l'eau présente une salinité totale de 30 à 300 g/litre.

**Patentansprüche**

1. Sulfobetain, dadurch gekennzeichnet, daß es der folgenden allgemeinen Formel entspricht:

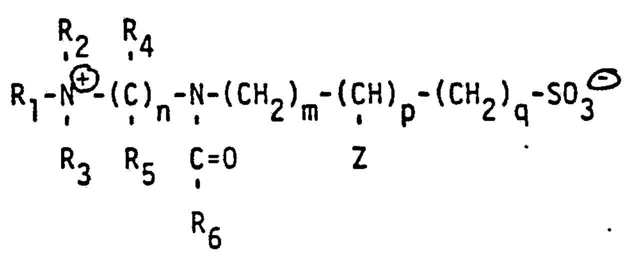

(I)

worin $R_1$, $R_2$ und $R_3$ jeweils einen linearen oder verzweigten aliphatischen, ggfs. hydroxylsubstituierten Rest, oder einen aromatischen oder arylaliphatischen Rest darstellen können, $R_4$ und $R_5$ jeweils ein Wasserstoffatom, eine Methylgruppe oder eine Hydroxymethylgruppe bedeuten und $R_6$ ein Wasserstoffatom, eine lineare oder verzweigte Alkylgruppe oder eine Arylgruppe sein kann, wobei die Gesamtheit der Reste $R_1$

bis $R_6$ 12 bis 30 Kohlenstoffatome enthält, n die ganze Zahl 2 oder 3 bedeutet, Z eine $CH_3$-Gruppe oder eine Hydroxylgruppe sein kann, p 0 oder 1 sein kann, wobei, wenn Z eine Hydroxylgruppe ist, p = 1 ist, m = 1 ist und q = 1 ist; wenn jedoch Z einen Methylrest bedeutet, ist p = 1, m = 2 und q kann den Wert 0 oder 1 haben, und wenn p = 0 ist, ist die Summe (m + q) = 2, 3 oder 4.

2. Sulfobetain nach Anspruch 1, dadurch gekennzeichnet, daß einer der drei Reste $R_1$, $R_2$ und $R_3$ zumindest 10 Kohlenstoffatome aufweist und die beiden anderen eine Kohlenstoffzahl von 1 oder 2 haben, $R_4$ und $R_5$ Wasserstoffatome sind und $R_6$ ein Wasserstoffatom, eine lineare oder verzweigte Alkylgruppe mit 1 bis 20 Kohlenstoffatomen oder eine Arylgruppe ist.

3. Sulfobetain nach Anspruch 1, dadurch gekennzeichnet, daß einer der drei Reste $R_1$, $R_2$ und $R_3$ zumindest 10 Kohlenstoffatome aufweist und die beiden anderen eine Kohlenstoffzahl von 1 oder 2 haben und $R_6$ ein Alkylrest mit 1 bis 6 Kohlenstoffatomen ist.

4. Sulfobetain nach einem der Ansprüche 1 bis 3, dadurch gekennzeichnet, daß $R_1$ ein gesättigter linearer aliphatischer Rest mit 12 bis 18 Kohlenstoffatomen ist, $R_2$ und $R_3$ Methylreste sind, n = 2 oder 3, p = 0 und (m + q) = 3 oder 4 sind.

5. Verfahren zur Herstellung eines Sulfobetains nach einem der Ansprüche 1 bis 4, dadurch gekennzeichnet, daß man in einer ersten Stufe ein intermediäres Sulfobetain der folgenden allgemeinen Formel herstellt:

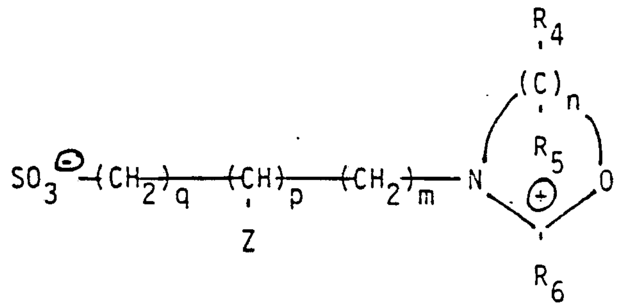

(IV)

worin $R_4$, $R_5$, $R_6$, n, m, p, q und Z die in Formel (I) definierten Bedeutungen haben und in einer zweiten Stufe man dieses intermediäre Sulfobetain mit einem tertiären Amin der folgenden allgemeinen Formel umsetzt:

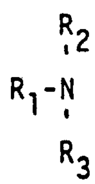

(V)

worin $R_1$, $R_2$ und $R_3$ die gleiche Definition wie in Formel (I) haben.

6. Verfahren nach Anspruch 5, dadurch gekennzeichnet, daß man in der ersten Stufe ein intermediäres Sulfobetain der allgemeinen Formel (IV) herstellt, worin Z ein Methylrest ist, wobei wenn p = 0 ist, m+q gleich 3 oder 4 ist und, wenn p = 1 ist, m = 2 und q = 0 oder 1 sind, indem man einen zyklischen Iminoäther der folgenden allgemeinen Formel

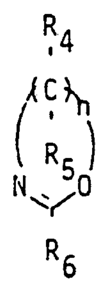

(II)

worin $R_4$, $R_5$, $R_6$ und n die in Formel (I) gegebene Definition haben, mit Propansulton, Butansulton, Methyl-3-propansulton oder Methyl-4-butansulton umsetzt.

7. Verfahren nach Anspruch 5, dadurch gekennzeichnet, daß man in der ersten Stufe ein intermediäres Sulfobetain der allgemeinen Formel (IV) herstellt, worin, wenn Z eine Hydroxylgruppe bedeutet, m, p und q jeweils den Wert 1 haben, indem man einen zyklischen Iminoäther der folgenden allgemeinen Formel

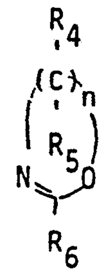

(II)

worin $R_4$, $R_5$, $R_6$ und n die in Formel (I) gegebene Definition haben, mit den Umsetzungsprodukt von Natriumbisulfit und Epichlorhydrin umsetzt.

8. Verfahren nach Anspruch 7, dadurch gekennzeichnet, daß man in der ersten Stufe ein intermediäres Sulfobetain der allgemeinen Formel (IV) herstellt, worin $p = 0$ und $m + q = 2$ sind, indem man ein Halogenwasserstoff oder ein Hydrogensulfat eines zyklischen Iminoäthers der folgenden allgemeinen Formel (IIa)

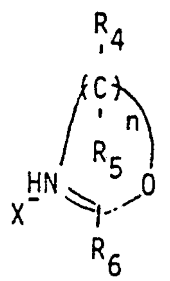

(IIa)

, worin X ein Hydrogensulfat- oder Halogenwasserstoffion bedeutet und $R_4$, $R_5$, $R_6$ und n die in Formel (I) gegebenen Definitionen haben, mit einem Ester der Äthensulfonsäure der Formel $CH_2 = CH - SO_3R$, worin R ein aliphatischer Rest mit 1 bis 10 Kohlenstoffatomen ist, reagieren läßt.

9. Verfahren nach Anspruch 5, dadurch gekennzeichnet, daß man in der ersten Stufe ein intermediäres Sulfobetain der allgemeinen Formel (IV) herstellt, worin $p = 0$ und $m + q = 3$ sind, indem man einen zyklischen Iminoäther der folgenden allgemeinen Formel

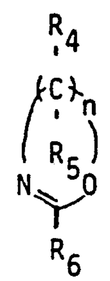

(II)

worin $R_4$, $R_5$, $R_6$ und n die in Formel (I) gegebenen Definitionen haben, mit Allylchlorid reagieren läßt und dann das erhaltenen Produkt mit Natriumbisulfit reagieren läßt.

10. Micellares System zur unterstützten Gewinnung von Erdöl aus Lagerstätten, dadurch gekennzeichnet, daß es als Tensid ein Sulfobetain nach einem der Ansprüche 1 bis 4 in Mischung mit Wasser, ggfs. mit zumindest einem flüssigen Kohlenwasserstoff und ggfs. mit zumindest einem Cotensid enthält.

11. Micellares System nach Anspruch 10, bestehend aus einer micellaren Lösung oder Mikroemulsion, dadurch gekennzeichnet, daß sie enthält:

- 70 bis 99,8 Gew.-% eines flüssigen Kohlenwasserstoffes und von Wasser in einem Gewichtsverhältnis flüssiger Kohlenwasserstoff/Wasser von 0 bis 4/1,
- 0,1 bis 15 Gew.-% Sulfobetain und
- 0 bis 15 Gew.-% Cotensid.

12. Micellares System nach einem der Ansprüche 10 und 11, dadurch gekennzeichnet, daß das Wasser einen Gesamtsalzgehalt von 30 bis 300 g/l aufweist.

**Claims**

1. A sulfobetaine characterized by the general formula

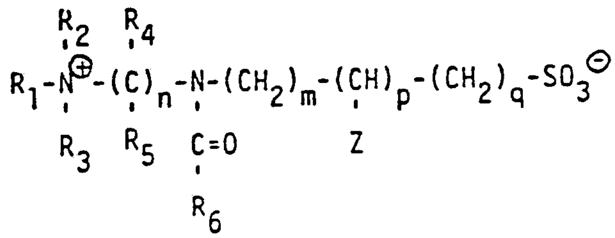

(I)

wherein each of $R_1$, $R_2$ and $R_3$ is a linear or branched aliphatic radical, carrying or not hydroxy groups, or aromatic or arylaliphatic radical, each of $R_4$ and $R_6$ is a hydrogen atom or a methyl or hydroxy-methyl group and $R_6$ is a hydrogen atom, a linear or branched alkyl group or an aryl group, $R_1$ to $R_6$ radicals containing together from 12 to 30 carbon atoms, n is an integer equal to 2 or 3, Z may be a $CH_3$ radical or a hydroxy group, p may be 0 or 1 with, when Z is a hydroxy group and p is equal to 1, a value of 1 for m and for Q; when Z is a methyl radical, and the value of p is 1, a value of 2 for m and a value of 0 or 1 for q and, when p=O, a value of 2, 3 or 4 for the sum (m + q).

2. A sulfobetaim according to claim 1, characterized in that one of the three radicals $R_1$, $R_2$ and $R_3$ contains at least 10 carbon atoms, the two others contain 1 or 2 carbon atoms, $R_4$ and $R_5$ are hydrogen atoms and $R_6$ is a hydrogen atom, a linear or branched alkyl group containing 1 to 20 carbon atoms or an aryl group.

3. Sulfobetaine according to claim 1, characterized in that one of the three radicals $R_1$, $R_2$ and $R_3$ contains at least 10 carbon atoms, the two others contain 1 or 2 carbon atoms and $R_6$ is in alkyl group containing 1 to 6 carbon atoms.

4. Sulfobetaine according to anyone of claims 1 to 3, characterized in that $R_1$ is a $C_{12}$-$C_{18}$ linear saturated aliphatic radical, $R_2$ and $R_3$ are methyl radicals, n is 2 or 3, p is 0 and (m + q) is equal to 3 or 4.

5. A process for manufacturing a sulfobetaine according to claim 1 characterized by a first step of preparing an intermediary sulfobetaine of general formula:

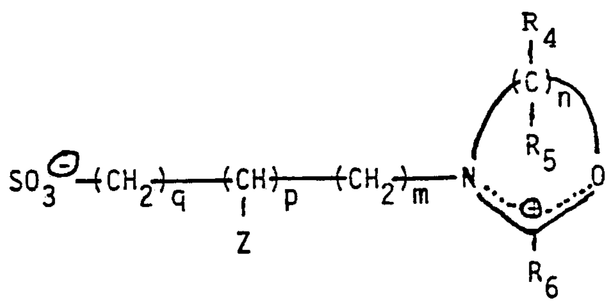

(IV)

wherein $R_4$, $R_5$, $R_6$, n, m, p, q and Z are defined as in formula 1, and by a second step of reacting said intermediary sulfobetaine with a tertiary amine of general formula:

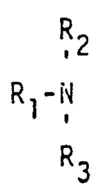

(V)

wherein $R_1$, $R_2$ and $R_3$ are defined as in formula (I).

6. A process according to claim 5, characterized in that the first step comprises preparing an intermediary sulfobetaine of general formula (IV) wherein Z is a methyl radical and, when p = 0, (m + q) is equal to 3 or 4 and, when p = 1, m is 2 and q is equal to 0 or 1, by reacting a cyclic iminoether of general formula:

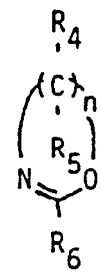

(II)

wherein $R_4$, $R_5$, $R_6$ and n are defined as in formula (I), with propane sultone, butane sultone, 3-methyl propane sultone or 4-methyl butane sultone.

7. A process according to claim 5, characterized by preparing, in the first step, an intermediary sulfobetaine of general formula (IV), wherein, when Z is hydroxy group, m, p, and q are each equal to 1, by reacting an iminoether of general formula:

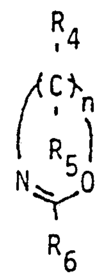

(II)

wherein $R_4$, $R_5$, $R_6$ and n are defined as in formula (I), with the reaction product of sodium bisulfite on epichlorhydrine.

8. A process according to claim 5, characterized in that, in the first step, an intermediary sulfobetaine of general formula (IV) is prepared, wherein p = 0 and (m + q) = 2, by reacting a cyclic iminoether halohydrate or hydrogenosulfate of general formula (IIa):

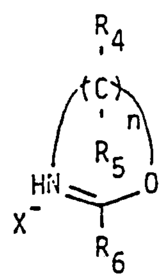

(IIa)

wherein X is a hydrogenosulfate or halohydrate ion, and where $R_4$, $R_5$, $R_6$ and n are defined as in formula (I), with an ester of ethylene sulfonic acid of formula $CH_2 = CH - SO_3R$, wherein R is an aliphatic radical comprising 1 to 10 carbon atoms.

9. A process according to claim 5, characterized by preparing, in the first step, an intermediary sulfobetaine of general formula (IV), wherein p = 0 and (m + q) = 3, by reacting a cyclic iminoether of general formula:

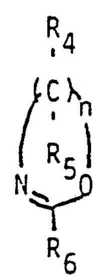

(II)

wherein $R_4$, $R_5$, $R_6$ and n are defined as in formula (I), with allyl chloride, and then reacting the resultant product with sodium bisulfite.

10. A micellar system for enhanced oil recovery from the fields, characterized in that it contains, as surfactant, a sulfobetaine according to one of claims 1 to 4, in admixture with water, optionally at least one hydrocarbon liquid and optionally at least one cosurfactant.

11. A micellar system according to claim 10, consisting of a micellar solution or a microemulsion, characterized in that it contains:

- from 70 to 99.9 % by weight of a hydrocarbon liquid and water in a ratio by weight hydrocarbon liquid/water from 0 to 4/1,
- from 0.1 to 15 % by weight of sulfobetaine and,
- from 0 to 15 % by weight of cosurfactant.

12. A micellar system according to anyone of claims 10 to 11 characterized in that water has a total salt content from 30 to 300 g/l.